# EUROPEAN PATENT APPLICATION

(11) **EP 4 506 469 A1**
(43) Date of publication of application: **12.02.2025**
(21) Application number: 23771153.6
(22) Date of filing: 20.03.2023
(51) Int. Cl.: C12Q 1/6883

(54) **BIOMARKER COMPOSITION FOR DIAGNOSING HEMOSTATIC DISORDERS, COMPRISING MIRNAS IN EXTRACELLULAR VESICLES**

(30) Priority: 18.03.2022 KR 20220033996
(71) Applicant: S&E Bio Co., Ltd., Seoul 06351 (KR); Samsung Life Public Welfare Foundation, Seoul 04348 (KR); Xenohelix. Co., Ltd., Incheon 21984 (KR)
(72) Inventor: BANG, Oh Young, Seoul 06277 (KR); KIM, Eun Hee, Seoul 06608 (KR); SUNG, Ji Hee, Seoul 08716 (KR); SHIN, Eun Kyoung, Seoul 07611 (KR); CHUNG, Jong Won, Seoul 06351 (KR); SEO, Woo Keun, Seoul 06351 (KR); KIM, Gyeong Moon, Seoul 06351 (KR); AHN, Myung Ju, Seoul 06351 (KR)
(74) Representative: Cabinet Becker et Associés
(86) International application number: PCT/KR2023/003670
(87) International publication number: WO 2023/177275

(57) **Abstract**

The present invention relates to biomarkers for diagnosing coagulation disorders, comprising miRNAs in extracellular vesicles. It has been experimentally identified that miRNAs in extracellular vesicles, according to the present invention, exhibit expression patterns in coagulation disorder patients that are more specific than those of normal groups. Therefore, it is indicated that miRNAs in extracellular vesicles of the present invention can be used as biomarkers of coagulation disorders, and thus the miRNAs can be variously used in the field of coagulation disorder diagnosis.

## Description

### [Technical Field]

The present invention relates to biomarkers for diagnosing coagulation disorders, including miRNAs in extracellular vesicles.

### [Background Art]

Among causes of death of patients with malignant tumors, in addition to the death due to cancer itself, coagulation disorders are caused by various procoagulants secreted by cancer cells and then death or disorder due to cerebral infarction or pulmonary embolism becomes a main cause of death or disorder in patients with malignant tumors. Various anticoagulants have been developed and used to effectively prevent and correct coagulation disorders. However, since there is no method to predict coagulation disorders in cancer patients, only after coagulation disorders occur, the coagulation disorders are diagnosed. In addition, since the anticoagulants have a risk of bleeding, it is difficult to recommend preventive administration in advance to cancer patients who have not developed thrombosis. Therefore, it is very important to predict the coagulation disorders in advance or diagnose the coagulation disorders quickly, but there is still no known effective preventive method that can predict the risk of blood coagulation in cancer patients in advance or diagnose the coagulation disorders early to prevent the coagulation disorders. Various clinical prediction methods, blood D-dimer levels, and the like are used in clinical practice, but have a non-specifically low diagnostic value.

Studies have been reported on diagnostic methods using microRNAs (miRNAs) in blood to predict prognosis including death, in patients with malignant tumors. However, cell-free miRNAs in blood are destroyed by RNases in blood, so that there is a need for a method for measuring more consistent miRNA levels.

Extracellular vesicles are classified into exosomes and microvesicles depending on a size, and the exosomes have a diameter of 30 to 150 nm, and the microvesicles have a size of 100 to 1,000 nm. The extracellular vesicles are parts of the cell membranes that have been separated into the blood, and are known to mediate intercellular communication by containing both proteins and nuclear components. The extracellular vesicles have been found to exist in most body fluids, such as blood, urine, saliva, breast milk, pleural fluid, ascites, and cerebrospinal fluid, which occur normally or during occurrence of diseases in the body, and thus clinical usefulness thereof is very high. In fact, efforts have been very actively made internationally to develop a method for diagnosing not only cancers but also various diseases by isolating and analyzing extracellular vesicles from body fluids. The miRNAs contained in the extracellular vesicles are protected from blood RNases by a lipid bilayer, and thus show higher and more consistent levels than cell-free miRNAs. It is known that miRNAs contained in the extracellular vesicles have different miRNA profiles between exosomes and microvesicles.

### [Disclosure]

### [Technical Problem]

Accordingly, the present inventors developed biomarkers for diagnosing coagulation disorders containing miRNAs in extracellular vesicles, and then completed the present invention.

An object of the present invention is to provide a biomarker composition for diagnosing coagulation disorders including at least one miRNA selected from the group consisting of miR-205-5p, miR-645, and miR-646.

Another object of the present invention is to provide a composition for diagnosing coagulation disorders including an agent for measuring the expression level of at least one miRNA selected from the group consisting of miR-205-5p, miR-645, and miR-646.

Yet another object of the present invention is to provide a kit for diagnosing coagulation disorders including the composition for diagnosing coagulation disorders.

Still another object of the present invention is to provide a method for providing information for diagnosing coagulation disorders, including (a) isolating extracellular vesicles from a biological sample of a target; and (b) comparing the expression level of at least one miRNA selected from the group consisting of miR-205-5p, miR-645, and miR-646 in the extracellular vesicles with the expression level of the corresponding miRNA in a comparative control sample.

Still another object of the present invention is to provide a method for diagnosing and treating coagulation disorders, including (a) obtaining an analysis sample from a subject, (b) diagnosing the subject with coagulation disorders when the expression level of at least one miRNA selected from the group consisting of miR-205-5p, miR-645, and miR-646 in the analysis sample of step (a) is highly expressed compared to a comparative control group, and (c) administering a coagulation disorder therapeutic agent in an effective amount to the subject diagnosed with coagulation disorders of step (b).

### [Technical Solution]

One aspect of the present invention provides a biomarker composition for use in diagnosing coagulation disorders including at least one miRNA selected from the group consisting of miR-205-5p, miR-645, and miR-646.

Another aspect of the present invention provides a composition for use in diagnosing coagulation disorders including an agent for measuring the expression level of at least one miRNA selected from the group consisting of miR-205-5p, miR-645, and miR-646.

Yet another aspect of the present invention provides a kit for use in diagnosing coagulation disorders including the composition for diagnosing coagulation disorders.

Still another aspect of the present invention provides a method for providing information for diagnosing coagulation disorders, including (a) isolating extracellular vesicles from a biological sample of a target; and (b) comparing the expression level of at least one miRNA selected from the group consisting of miR-205-5p, miR-645, and miR-646 in the extracellular vesicles with the expression level of the corresponding miRNA in a comparative control sample.

Still another aspect of the present invention provides a method for diagnosing and treating coagulation disorders, including (a) obtaining an analysis sample from a subject, (b) diagnosing the subject with coagulation disorders when the expression level of at least one miRNA selected from the group consisting of miR-205-5p, miR-645, and miR-646 in the analysis sample of step (a) is highly expressed compared to a comparative control group, and (d) administering a coagulation disorder therapeutic agent in an effective amount to the subject diagnosed with coagulation disorders of step (c).

### [Advantageous Effects]

According to the present invention, it has been experimentally identified that miRNAs in extracellular vesicles exhibit expression patterns in coagulation disorder patients that are more specific than those of normal groups. Therefore, it is indicated that miRNAs in extracellular vesicles of the present invention can be used as biomarkers of coagulation disorders, and thus the miRNAs can be variously used in the field of coagulation disorder diagnosis.

### [Description of Drawings]

FIG. 1 is a diagram illustrating an overall research design.
FIG. 2A is a diagram illustrating an isolation process of microvesicles and exosomes among extracellular vesicles in a patient's blood.
FIG. 2B is a diagram illustrating a result of identifying a size distribution of extracellular vesicles through NanoSight Tracking Analysis.
FIG. 2C is a diagram illustrating a result of identifying the morphology of extracellular vesicles using an electron microscope.
FIG. 2D is a diagram illustrating a result of detecting positive and negative markers of extracellular vesicles through Western blotting.
FIG. 3 is a diagram illustrating a result of deriving miRNAs candidates associated with cancer-related coagulation disorders by measuring miRNA levels of blood microvesicles and exosomes in pooling samples from a patient group with cerebral infarction and a patient group without cerebral infarction among cancer patients (CC : cancer-control, CS : cancer-stroke).
FIG. 4A is a diagram illustrating a result of comparing miRNA levels in microvesicles in each sample from a normal group, a cancer patient group without cerebral infarction, and a cancer patient group with cerebral infarction.
FIG. 4B is a diagram showing receiver operating characteristics (ROC) curves for cerebral infarction (cancer-stroke) due to cancer-related coagulation disorders among cancer patients, based on the result of comparing the miRNA levels in microvesicles in each sample.
FIG. 5A is a diagram illustrating results of comparing miR-205-5p, miR-645, and miR-646 levels in microvesicles in groups of patients with cerebral infarction due to cancer-related coagulation disorders (cancer-stroke) and cerebral infarction without cancer (stroke-control, acute stroke) among stroke patients.
FIG. 5B is a diagram showing receiver operating characteristics (ROC) curves for cerebral infarction due to cancer-related coagulation disorders (cancer-stroke) among cancer patients, based on the result of comparing the miR-205-5p, miR-645 and miR-646 levels in microvesicles in each sample.
FIG. 6 is a diagram illustrating results (A and B) of comparing the expression of mRNA using a Xeno-sensor technique in an independent cohort for external validation to diagnose cerebral infarction due to cancer-related coagulation disorders, and a result (C) of diagnosing cerebral infarction due to cancer-related coagulation disorders.
FIG. 7A is a diagram illustrating a result of identifying functions of target genes of increased miRNAs in microvesicles in patients with cerebral infarction due to blood cancer-related coagulation disorders through gene ontology (GO) analysis.
FIG. 7B is a diagram illustrating a result of identifying functions of target genes of increased miRNAs in microvesicles in patients with cerebral infarction due to cancer-related coagulation disorders in the blood through Kyoto Encyclopedia of Genes and Genomes (KEGG) analysis.
FIG. 7C is a diagram illustrating a result of analyzing mRNA expression of VEGFA according to infection with con-miR, pre-miR-205-5p, pre-miR-645, or pre-miR-646 in human umbilical vein endothelial cells.
FIG. 7D is a diagram illustrating a result of analyzing protein expression of VEGFA according to infection with con-miR, pre-miR-205-5p, pre-miR-645, or pre-miR-646 in human umbilical vein endothelial cells.

### [Best Mode of the Invention]

Hereinafter, the present invention will be described in detail.

According to an aspect of the present invention, there is provided a biomarker composition for diagnosing coagulation disorders including at least one miRNA selected from the group consisting of miR-205-5p, miR-645, and miR-646.

In a specific embodiment of the present invention, the miR-205-5p may be represented by a base sequence represented by SEQ ID NO: 1 (UCCUUCAUUCCACCGGAGUCUG), the miR-645 may be represented by a base sequence represented by SEQ ID NO: 2 (UCUAGGCUGGUACUGCUGA), and the miR-646 may be represented by a base sequence represented by SEQ ID NO: 3 (AAGCAGCUGCCUCUGAGGC).

That is, the greatest feature of the present invention is to diagnose and predict the onset of coagulation disorders by using the miRNA as a biomarker.

As used herein, the term "miRNA (microRNA)" refers to untranslated RNA of approximately 22 nt that acts as a post-transcriptional repressor through base binding to a 3' untranslated region (UTR) of mRNA.

As used herein, the "diagnosis" means identifying the presence or characteristics of pathological conditions. The diagnosis includes identifying not only the onset of the disease, but also the prognosis, course, and stage of the disease. For the purposes of the present invention, the diagnosis means identifying the onset, prognosis, course, stage or characteristics of coagulation disorders.

As used herein, the "biomarker" may be used as a diagnostic marker and refers to a substance that may separately diagnose whether or not coagulation disorders occur in a biological sample.

In a specific embodiment of the present invention, the coagulation disorders are preferably cancer-related coagulation disorders, and more preferably, the cancer-related coagulation disorders may be cancer-related stroke, but are not limited thereto. The cancer-related coagulation disorders refer to coagulation disorders caused by various procoagulants secreted from cancer cells, and a representative example of cancer-related coagulation disorders is stroke.

In a specific embodiment of the present invention, the miR-205-5p, miR-645, and miR-646 may be miRNAs within extracellular vesicles, and preferably miRNAs present within extracellular vesicles in blood.

In a preferred embodiment of the present invention, the extracellular vesicles are preferably microvesicles.

As used herein, the "extracellular vesicles (EVs)" are classified into exosomes and microvesicles depending on a size, and the exosomes have a diameter of 30 to 150 nm, and the microvesicles have a size of 100 to 1,000 nm. MiRNAs contained in the extracellular vesicles are protected from blood RNases by a lipid bilayer, and thus show higher and more consistent levels than cell-free miRNAs. In addition, it is known that miRNAs contained in the extracellular vesicles have different miRNA profiles between exosomes and microvesicles.

In a specific embodiment of the present invention, it is preferred that the miR-205-5p, miR-645 and miR-646 are highly expressed when coagulation disorders occur. More specifically, the expression of miR-205-5p, miR-645, and miR-646 is significantly increased in a patient group with the cancer-related coagulation disorders compared to a normal group, a cancer patient group without stroke and a stroke group without cancer, and thus can be used as biomarkers of cancer-related coagulation disorders.

As used herein, the term "high expression" used when referring to the expression level of miRNA refers to a value or level of a biomarker in a biological sample that is higher than a value or level range of a biomarker detected in a biological sample obtained from a healthy or normal subject or a comparative subject, when the biomarker shows an abnormal process, a disease or other pathologies in the subject or has symptoms thereof.

According to another aspect of the present invention, there is provided a composition for diagnosing coagulation disorders including an agent for measuring the expression level of at least one miRNA selected from the group consisting of miR-205-5p, miR-645, and miR-646.

As used herein, the "agent for measuring the expression level of miRNA" refers to a molecule that may be used for detection of markers by specifically binding to miR-205-5p, miR-645, and miR-646, which are markers with increased expression in biological samples of patients with coagulation disorders to identify the expression levels thereof, as described above.

Unless otherwise specified herein, the expression "measuring the expression level of miRNA" used herein means detecting a target to be detected within the corresponding sample.

In a specific embodiment of the present invention, the agent may be a primer or probe that specifically binds to at least one miRNA selected from the group consisting of miR-205-5p, miR-645, and miR-646.

As used herein, the term "primer" refers to a nucleic acid sequence having a short free 3' hydroxyl group, and a short nucleic acid sequence capable of forming a base pair with a complementary template and serving as a starting point for copying a template strand. The primer may initiate DNA synthesis in the presence of a reagent for polymerization (i.e., DNA polymerase or reverse transcriptase) and four different nucleoside triphosphates in appropriate buffer and temperature. Specifically, coagulation disorders may be diagnosed by performing PCR amplification using sense and antisense primers of TrioBP polynucleotide and determining whether a desired product is generated. The PCR conditions, and the lengths of sense and antisense primers may be modified based on those known in the art.

As used herein, the term "probe" refers to a nucleic acid fragment such as RNA or DNA corresponding to several bases in short to several hundred bases in length capable of forming a specific binding to mRNA, and is labeled to identify the presence or absence of specific mRNA. The probe may be prepared in the form of an oligonucleotide probe, a single stranded DNA probe, a double stranded DNA probe, an RNA probe, or the like. Selection of an appropriate probe and hybridization conditions may be modified based on those known in the art.

The primer or probe of the present invention may be chemically synthesized using a phosphoramidite solid support method, or other well-known methods. Such a nucleic acid sequence may also be modified using many means known in the art. Non-limiting examples of such a modification include methylation, "encapsulation", substitution with one or more homologs of natural nucleotides, and modifications between nucleotides, for example, modification to uncharged linkers (e.g., methyl phosphonate, phospholister, phosphoroamidate, carbamate, etc.) or charged linkers (e.g., phosphorothioate, phosphorodithioate, etc.).

The expression level of miRNA may be measured by methods commonly used in a bio-kit field, for example, reverse transcriptase polymerase reaction (RT-PCR), competitive RT-PCR, real-time RT-PCR, RNase protection assay (RPA), Northern blotting, or gene chips, but are not limited thereto.

According to yet another aspect of the present invention, there is provided a kit for diagnosing coagulation disorders including the composition for diagnosing coagulation disorders.

The kit may include not only an agent for measuring the expression level of the exosomal miRNA, but also tools, reagents, etc. commonly used in the art suitable for being used as a diagnostic kit for coagulation disorders.

Examples of the tool or reagent include suitable carriers, labeling substances capable of generating a detectable signal, chromophores, solubilizers, detergents, buffers, stabilizers, and the like, but are not limited thereto. If the labeling substance is an enzyme, the labeling substance may include a substrate capable of measuring enzyme activity and a reaction stopper. The carrier may be a soluble carrier and an insoluble carrier, and an example of the soluble carrier is a physiologically acceptable buffer known in the art, for example, PBS, and an example of the insoluble carrier may be polymers such as polystyrene, polyethylene, polypropylene, polyester, polyacrylonitrile, fluorine resin, cross-linked dextran, polysaccharide, metal-plated magnetic microparticles on latex, other paper, glass, metal, agarose, and combinations thereof.

For example, the diagnostic kit of the present invention may be a kit including essential elements required for performing RT-PCR. The RT-PCR kit may include test tubes or other suitable containers, reaction buffers (having various pHs and magnesium concentrations), deoxynucleotides (dNTPs), enzymes such as Taq-polymerase and reverse transcriptase, DNAse, RNAse inhibitor, DEPC-water, sterilized water, etc., in addition to each primer pair specific to marker miRNA.

Since the kit of the present invention includes the above-described biomarker and composition as a configuration, the description of duplicated contents will be omitted in order to avoid excessive complexity of the present specification.

According to still another aspect of the present invention, there is provided a method for providing information for diagnosing coagulation disorders, including (a) isolating extracellular vesicles from a biological sample of a target; and (b) comparing the expression level of at least one miRNA selected from the group consisting of miR-205-5p, miR-645 and miR-646 in the extracellular vesicles with the expression level of the corresponding miRNA in a comparative control sample.

In a specific embodiment of the present invention, the method may further include measuring the expression level of at least one miRNA selected from the group consisting of miR-205-5p, miR-645, and miR-646.

In a specific embodiment of the present invention, step (b) is preferably determining that the target is coagulation disorders when the expression level of at least one miRNA selected from the group consisting of miR-205-5p, miR-645, and miR-646 is highly expressed compared to a comparative control group.

As used herein, the "biological sample" means any sample obtained from a subject from which expression of the biomarker of the present invention may be detected.

In a specific embodiment of the present invention, the biological sample is blood or serum, and may be treated and prepared by a method commonly used in the technical field of the present invention, but is not particularly limited thereto.

Unless otherwise specified herein, the term "control group" as used herein refers to the above-described miRNA expression levels in samples from a normal group, a cancer patient group without stroke, and a stroke group without cancer.

In the method, if the expression level of at least one miRNA selected from the group consisting of miR-205-5p, miR-645 and miR-646 described above is measured to be significantly higher in samples from patients suspected of having the onset or progression of coagulation disorders than the expression levels of miR-205-5p, miR-645 and miR-646 in the control sample, information that coagulation disorders have occurred may be provided. In particular, the biomarkers miR-205-5p, miR-645 and miR-646 used in the method are highly expressed in a cancer-related stroke patient group compared to comparative control groups (normal group, cancer patient group without stroke, and stroke group without cancer), and thus may provide information on the diagnosis of cancer-related coagulation disorders.

According to still another aspect of the present invention, there is provided a method for diagnosing and treating coagulation disorders, including (a) obtaining an analysis sample from a subject, (b) diagnosing the subject with coagulation disorders when the expression level of at least one miRNA selected from the group consisting of miR-205-5p, miR-645, and miR-646 in the analysis sample of step (a) is highly expressed compared to a comparative control group, and (c) administering a coagulation disorder therapeutic agent in an effective amount to the subject diagnosed with coagulation disorders of step (b).

In the present invention, the coagulation disorder therapeutic agent refers to an agent for alleviating or treating the symptoms of coagulation disorders.

In a specific embodiment of the present invention, the coagulation disorder therapeutic agent is preferably at least one selected from the group consisting of heparin, dalteparin, enoxaparin, tanzapirin, warfarin, xarelto, eliquis, pradaxa, and Lixiana, but is not limited thereto.

Among the coagulation disorder therapeutic agents, the dalteparin, enoxaparin, and tanzapirin belong to low molecular weight heparin (LMWH), and the warfarin belongs to coumarin-based drugs (vitamin K antagonist, coumarins). In addition, the xarelto, eliquis, pradaxa, and Lixiana belong to new oral anticoagulants (NOACs).

Duplicated contents are omitted in consideration of the complexity of the present specification, and terms not defined otherwise in the present specification have the meanings commonly used in the art to which the present invention pertains.

### [Modes of the Invention]

Hereinafter, the present invention will be described in more detail through Examples. These Examples are just illustrative of the present invention, and it will be apparent to those skilled in the art that it is not interpreted that the scope of the present invention is limited to these Examples.

### Example 1. Collection of extracellular vesicles in blood in clinical trial groups

### 1.1. Setting of clinical trial groups

Serum samples were obtained from a total of 220 patients targeted in an OASIS-CANCER clinical trial (Clinicaltrials.gov. Unique identifier: NCT02743052). The trial participants included 45 patients with cancer-related cerebral infarction (cancer-stroke), and 76 normal persons (healthy-controls), 39 cancer patients without stroke (cancer-controls), and 60 cerebral infarction patients without cancer (stroke-controls) as control groups.

The cancer-related cerebral infarction group (cancer-stroke) was a case in which cerebral infarction occurred in patients diagnosed with a malignant tumor or receiving anticancer treatment within the past 6 months, and in which there was no cause other than cancer-related embolism, such as arteriosclerosis or cardioembolism. The normal group (healthy-control) was a patient group who has not been diagnosed with stroke or malignant tumor, the cancer patient group without stroke (cancer-control) was a patient group who has been diagnosed with a malignant tumor or has received anticancer treatment within the past 6 months to have no history of stroke, and the cerebral infarction group without cancer (stroke-control) was a case that developed due to common cerebral infarction mechanisms such as arteriosclerosis or cardioembolism (CE).

A developing cohort for calculating biomarker candidates consisted of 143 patients, and a validation cohort included 77 patients as an independent patient group for validating biomarker candidates. In both the developing cohort and the validation cohort, the stroke-control group was older than the other groups. In both the cancer-control and cancer-stroke groups, lung cancer was the most common primary cancer, and mostly metastasized. Information on the trial participants was shown in Tables 1 and 2.

**[Table 1]**

| | Healthy-control (n = 48) | Cancer-control (n = 23) | Stroke-control (n = 40) | Cancer-stroke (n = 32) | P-value |
|---|---|---|---|---|---|
| Age | 57.9 ± 9.8 | 60.0 ± 9.5 | 75.0 ± 9.2 | 65.3 ± 11.8 | < 0.001 |
| Male | 14(29.2%) | 12(52.2%) | 17(42.5%) | 15(46.9%) | 0.209 |
| Risk factor | | | | | |
| Hypertension | 14(29.2%) | 7(30.4%) | 32(80.0%) | 11(34.4%) | < 0.001 |
| Diabetes | 5(10.4%) | 4(17.4%) | 16(40.0%) | 6(18.8%) | 0.010 |
| Dyslipidemia | 5(10.4%) | 3(13.0%) | 11(27.5%) | 3(9.4%) | 0.128 |
| Smoking | 7(14.6%) | 5(21.7%) | 3(7.5%) | 3(9.4%) | 0.374 |
| Prior stroke | - | - | 10(25.0%) | 8(25.0%) | < 0.001 |
| Systemic cancer | | | | | |
| Lung cancer | - | 20(87.0%) | - | 13(40.6%) | |
| Metastasis | - | 15(65.2%) | | 28(87.5%) | |

**[Table 2]**

| | Healthy-control (n = 28) | Cancer-control (n = 16) | Stroke-control (n = 20) | Cancer-stroke (n = 13) | P-value |
|---|---|---|---|---|---|
| Age | 59.3 ± 5.5 | 62.6 ± 8.2 | 68.3 ± 14.0 | 64.6 ± 7.6 | 0.013 |
| Male | 10(35.7%) | 9(56.2%) | 15(75.0%) | 4(30.8%) | 0.024 |
| Risk factor | | | | | |
| Hypertension | 8(28.6%) | 5(31.2%) | 13(65.0%) | 4(30.8%) | 0.061 |
| Diabetes | 3(10.7%) | 1(6.2%) | 6(30.0%) | 1(7.7%) | 0.193 |
| Dyslipidemia | 3(10.7%) | 2(12.5%) | 10(50.0%) | 2(15.4%) | 0.010 |
| Smoking | 4(14.3%) | 9(56.2%) | 5(25.0%) | 1(7.7%) | 0.010 |
| Prior stroke | - | - | 5(25.0%) | 2(15.4%) | < 0.001 |
| Systemic cancer | | | | | |
| Lung cancer | - | 16(100.0%) | - | 6(46.2%) | |
| Metastasis | - | 13(81.2%) | | 10(76.9%) | |

### 1.2 Collection and analysis of extracellular vesicles in blood

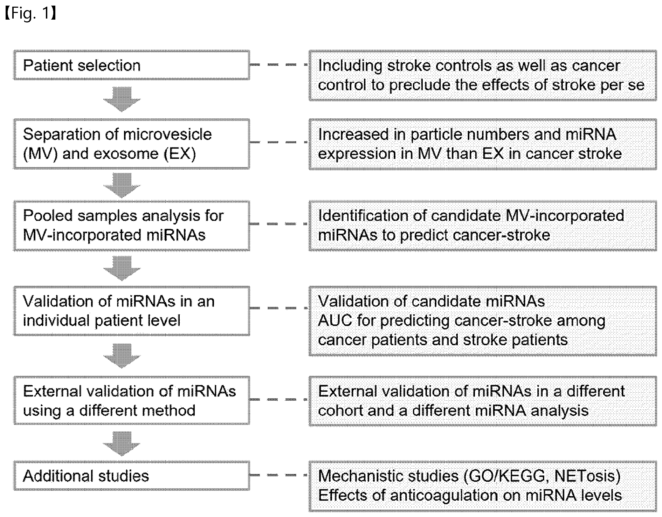

The overall clinical trial design was presented in FIG. 1.

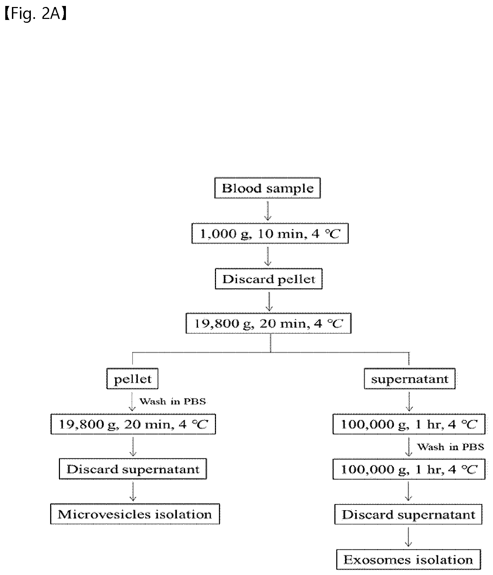

In all patients, plasma samples were collected from the veins. Plasma samples were prepared from citrated whole blood (citrate plasma), and then immediately centrifuged at 2000 g for 15 minutes, and stored at - 80°C for experiments. Microvesicles and exosomes were isolated using a differential ultra-centrifugation method. The centrifugation process was performed using Optima TLX ultracentrifuge (Beckman Coulter, Brea, CA, USA) and TLA120.2 rotor at 4°C. The specific method was presented in FIG. 2A.

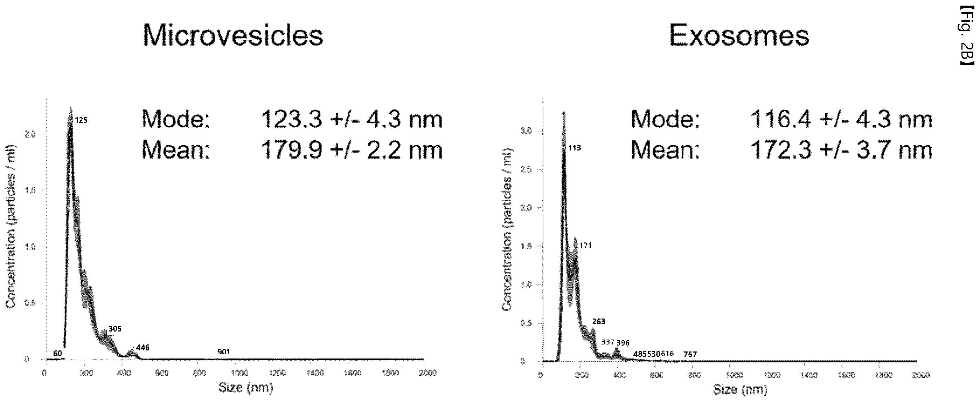

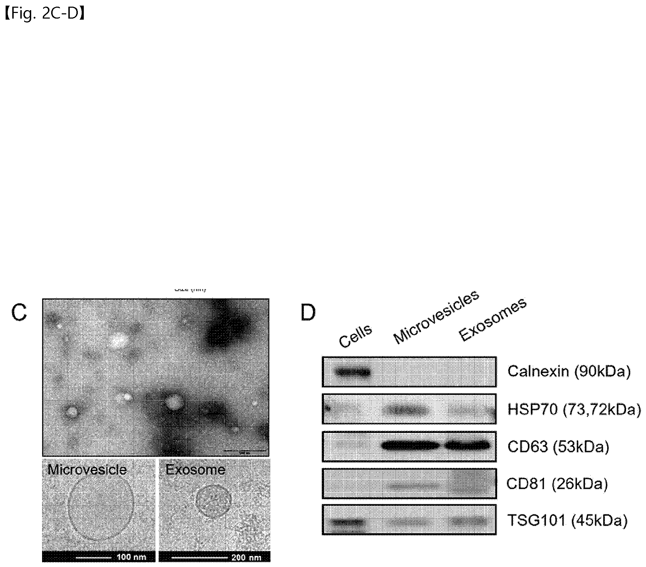

For optimal analysis, extracellular vesicles were pre-diluted in vesicle-free water, and the concentration and size distribution of the extracellular vesicles were measured using a NanoSight NS300 instrument (Malvern, Worcestershire, UK). The mean size and concentration (particles/mL) of the extracellular vesicles were calculated by integrating three individual measurement data. The extracellular vesicles were directly visualized using transmission electron microscopy (TEM). To identify positive and negative markers of extracellular vesicles, 20 µg of extracellular vesicle proteins were separated by SDS-PAGE and transferred to a nitrocellulose membrane (Bio-Rad, Hercules, CA, USA). The membrane was incubated overnight at 4°C with primary antibodies against CD63, Flotillin-1, and HSP70 (1:1000, Cell Signaling Technology, Beverly, MA, USA), or calnexin (1:1000, Santa Cruz Biotechnology, Santa Cruz, CA, USA), and then reacted with a horseradish peroxidase (HRP)-conjugated secondary antibody (1:1000, anti-rabbit, Cell Signaling Technology) for 2 hours. The proteins were detected using a chemiluminescence substrate from ThermoFisher Scientific, Inc. (Waltham, MA, USA) and visualized with an X-ray film (Agfa, Mortsel, Belgium). After MSC treatment, the levels of plasma extracellular vesicles were measured in patients, and the shape and size distribution of the plasma extracellular vesicles were identified through Cryo-TEM, and the results were illustrated in FIGS. 2B and 2C, and the results of detecting the positive and negative markers of the extracellular vesicles were illustrated in FIG. 2D.

As illustrated in FIGS. 2B and 2C, it was identified that most extracellular vesicles had a round shape in an electron-dense structure, and the mean (SD) diameters were 179.9 ± 2.2 nm for microvesicles and 172.3 ± 3.7 nm for exosomes, respectively.

As illustrated in FIG. 2D, circulating extracellular vesicles were positive for extracellular vesicles markers including CD63, CD81, TSG101 and HSP70, and as a negative marker for extracellular vesicles, Calnexin was not detected.

### Example 2. Analysis of miRNA level in extracellular vesicles

### 2.1 Derivation of miRNA candidates for cancer-related cerebral infarction in blood microvesicles and exosomes

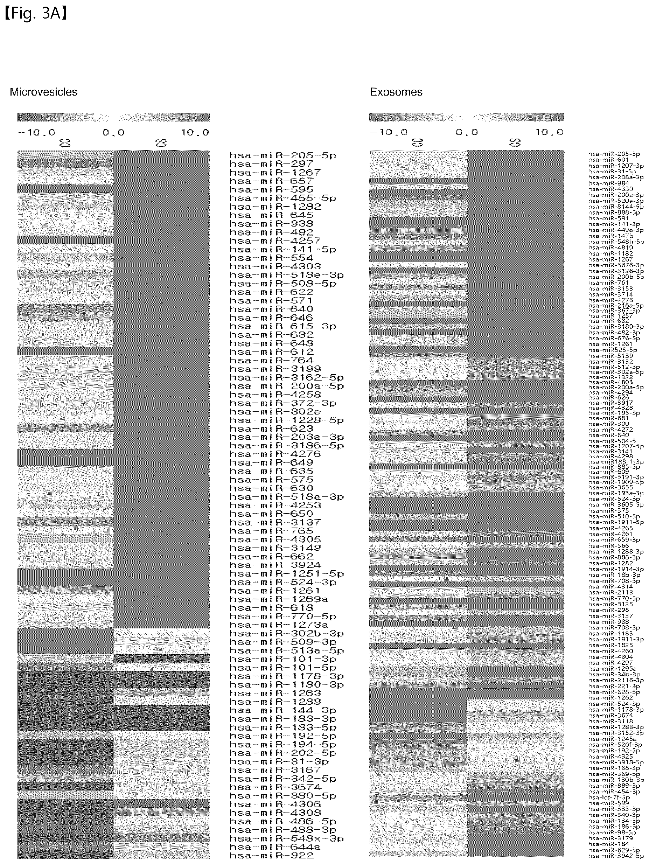

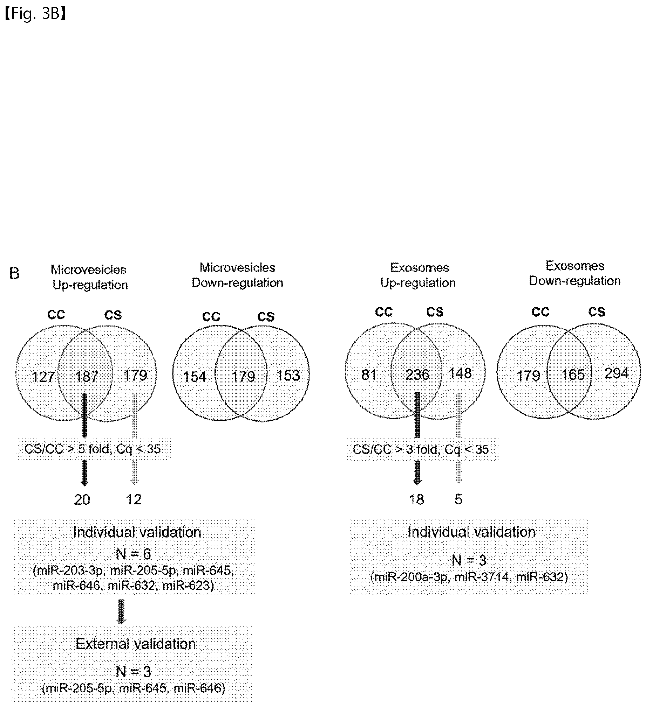

After collecting sera from healthy-control, cancer-control, and cancer-stroke groups, the miRNA levels in pooled samples were compared between microvesicles and exosomes using microarrays. Specifically, miRNA profiles were measured using the Human whole-miRNome Array and a miScript SYBR green PCR kit. 4 µl RNA was reverse transcribed into cDNA using a mi Script II RT kit. A PCR template was used at a 1:20 dilution of a RT product, and qRT-PCR was performed in 384 wells containing synthetic miRNAs miRTC, a positive PCR control group, and a housekeeping reference using the ABI 7900HT Real-Time PCR System. Raw data were normalized to the reference miRNA included in all plates and normalized to the Global CT mean to calculate Cq values. The microarray results were illustrated in FIGS. 3A and 3B.

As illustrated in FIG. 3A, it was identified that each group had a difference in expression level of miRNA between microvesicles and exosomes.

As illustrated in FIG. 3B, in the case of microvesicles, among miRNAs with increased expression in the cancer-stroke group, there were 32 miRNAs with 5-fold or higher increased expression compared to that of the cancer-control group and Cq values of less than 35, and as a result identified with each individual patient sample, 7 miRNAs had increased expression in the cancer-stroke. Among these, only three miRNAs, that is, miR-205-5p, miR-645, and miR-646, were increased compared to the stroke control, which was identified that the increased expression was not caused by stroke.

In the case of exosomes, 23 miRNAs were increased 3-fold or higher in the cancer-stroke group compared to the cancer-control group and had Cq values of less than 35. However, the expression levels of these miRNAs were not different from those of the stroke-control group. Therefore, in the case of microvesicles, three miRNAs showed a significant difference in expression in the cancer-stroke group, whereas in the case of exosomes, miRNAs showed no significant difference in expression, so that subsequent studies were conducted on miRNA analysis in microvesicles.

### 2.2 Derivation of miRNA candidates for cancer-related cerebral infarction in blood microvesicles

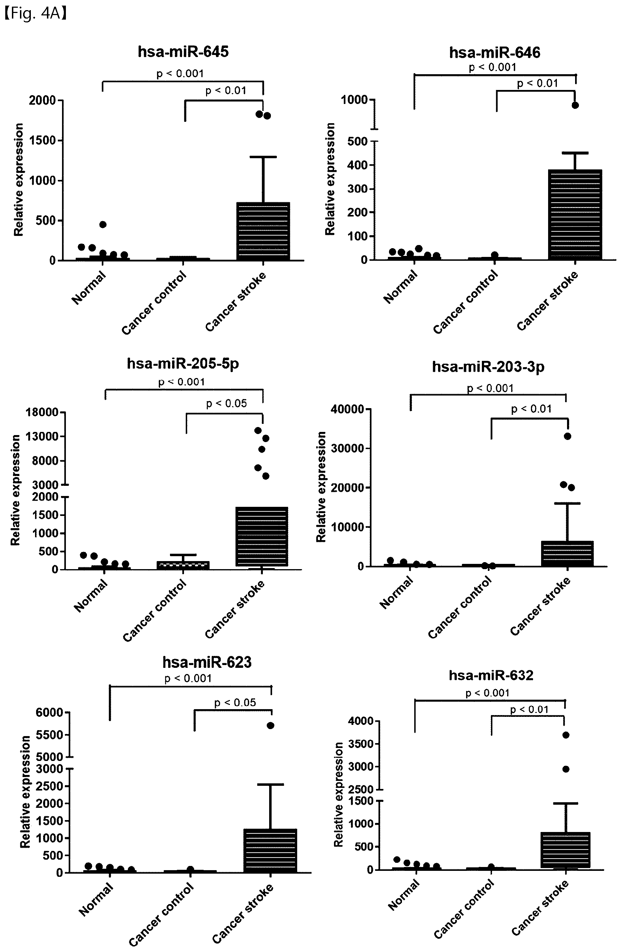

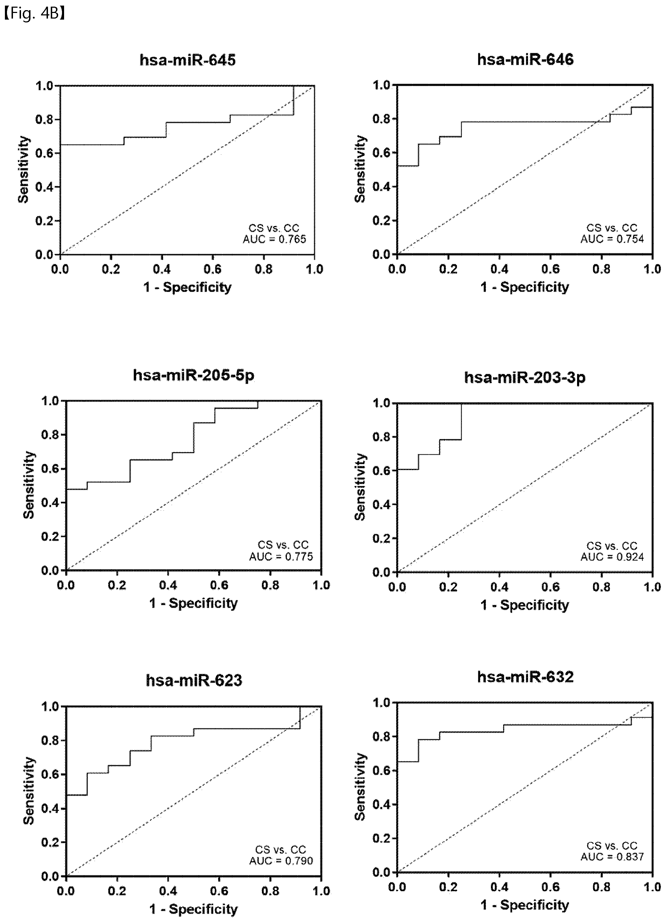

The expression levels of miRNAs in microvesicles in individual patient samples were compared for each group using qPCR. The expression levels were measured using TaqMan MicroRNA Assays (Applied Biosystems, Foster City, CA, USA) primers and analyzed by a 2-ΔCt quantitative method. MiRNAs with Cq values of less than 35 and 5-fold or higher increased expression compared to the healthy control were selected. The results of comparing the expression levels of miRNAs in microvesicles in individual patient samples were illustrated in FIG. 4.

As illustrated in FIG. 4, miR-203-3p, miR-205-5p, miR-645, miR-646, miR-632, and miR-623 contained in microvesicles were significantly increased in cancer stroke compared to healthy-control or cancer-control (FIG. 4A), and these miRNAs showed various differential diagnostic powers with AUC of 0.754 to 0.924 (FIG. 4B).

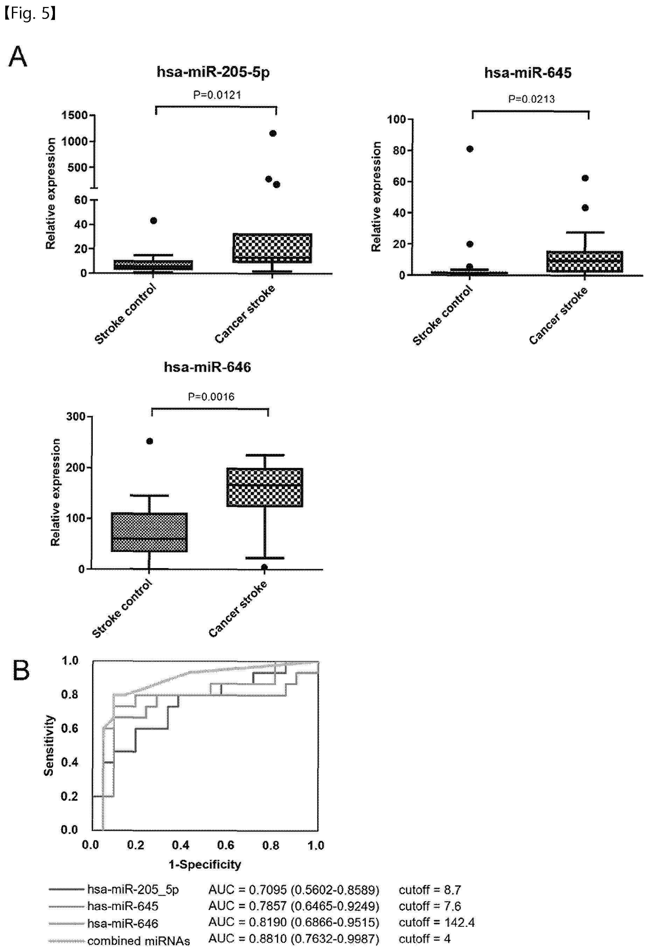

The miRNA levels in microvesicles in the cancer-stroke and stroke-control groups among stroke patients were compared with each other, and the results were illustrated in FIG. 5.

As illustrated in FIG. 5, miR-205-5p, miR-645, and miR-646 showed significantly higher expression levels than the stroke-control (FIG. 5A), and had the AUC of 0.7095 to 0.8190, and showed differential diagnostic power in cases of cerebral infarction. When three miRNAs were combined, the differential diagnostic power increased to 0.8810 (FIG. 5B).

The miR-205-5p, miR-645, and miR-646 were represented by base sequences of SEQ Nos: 1 to 3, respectively.

### 2.3 Validation of miRNAs in blood microvesicles for cancer-related cerebral infarction

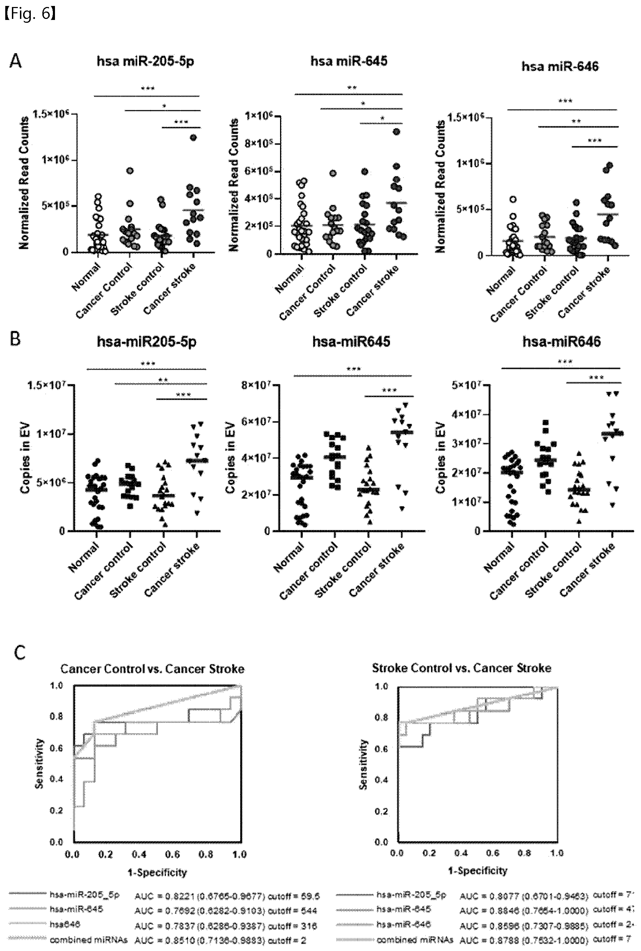

For external validation, the expression levels of miR-205-5p, miR-645, and miR-646 in microvesicles in individual patient samples were compared using the Xeno-sensor technique in independent patients. The results of analyzing the mRNA expression using the xeno-sensor technique were illustrated in FIG. 6.

As illustrated in FIGS. 6A and 6B, it was identified that miR-205-5p, miR-645, and miR-646 in extracellular vesicles and microvesicles were significantly higher expressed in the cancer-stroke than the healthy-control (Normal), cancer-control, and stroke-control (CE-AF). In addition, as a result of quantitative evaluation of miRNAs, it was identified that the copy number of each miRNA was significantly different.

As illustrated in FIG. 6C, in the accuracy of diagnosing the occurrence of cerebral infarction in cancer patients, miR-205-5p, miR-645, and miR-646 had the AUC of 0.8221, 0.7692, and 0.7837, respectively, and when the three miRNAs were combined, the AUC was 0.8510. In the diagnostic accuracy for differentiating cerebral infarction patients due to cancer-related coagulation disorders, the AUC were 0.8077, 0.8846, and 0.8596, respectively. When three miRNAs were combined, the AUC was 0.8788.

### 2.4 Bioinformatics analysis of miRNA

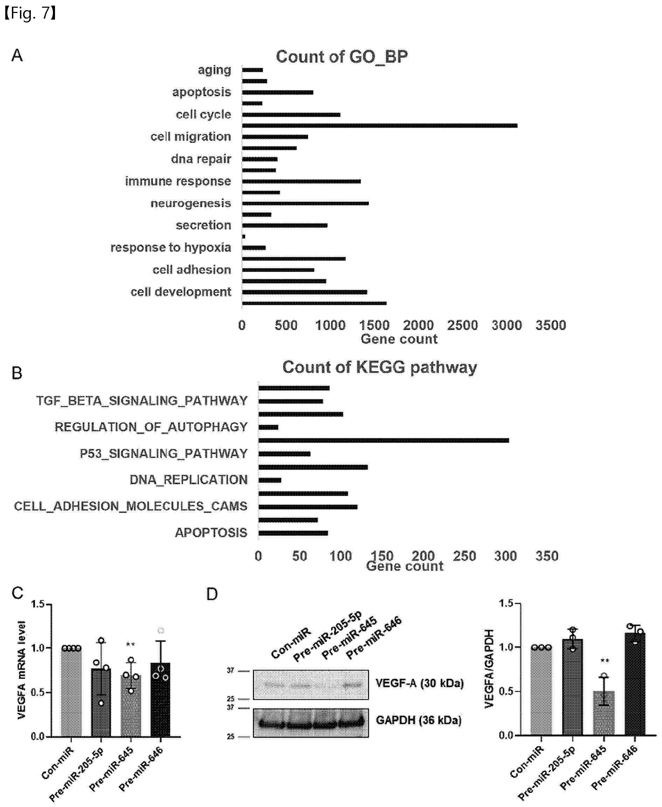

Bioinformatics analysis of miRNAs was performed. Specifically, after ensuring whether data were properly normalized (miRNA profiles were analyzed using Human whole-miRNome Array), miRNAs that showed a 2.0-fold or higher difference between the mean signal values of Healthy Individual (HC), Cancer-Control (CC), and Cancer-Stroke (CS) groups were selected. To classify co-expression of miR groups with similar expression patterns, hierarchical clustering analysis was performed using Multi Experiment Viewer (MEV) software version 4.9.0. The functions of differentially expressed miRNAs selected from target genes were detected using Kyoto Encyclopedia of Genes and Genomes (KEGG) and Gene Ontology (GO) pathway analysis. GO and KEGG pathway enrichment analysis were performed by using a miRWalk2.0 web-based tool (//zmf.umm.uni heidelberg.de/apps/zmf/mirwalk2/). Potential miRNA targets were predicted based on an RNA22 database (//cm.jefferson.edu/rna22). The results of bioinformatics analysis of miRNAs were illustrated in FIGS. 7A and 7B.

As illustrated in FIGS. 7A and 7B, GO and KEGG analysis results showed that miRNAs in microvesicles with increased expression in cancer stroke patients were related to cancer pathways; JAK/STAT signaling pathways; signaling pathways related to cell adhesion, cell cycle, cell differentiation, neural regeneration, and immune response; and biological pathways. In particular, three miRNAs in microvesicles interacted with target genes related to cell-matrix adhesion, CNS development, and positive regulation of peptidyl-serine phosphorylation.

### 2.5 Expression of vascular endothelial growth factor A (VEGFA) mRNA according to infection with three miRNAs

To confirm the regulation of VEGFA by three miRNAs, human umbilical vein endothelial cells (HUVECs) were transfected with con-miR, pre-miR-205-5p, pre-miR-645, or pre-miR-646. Specifically, the HUVECs were cultured in an EBMTM-2 basal medium (Lonza, Cat# CC-3156) supplemented with an EGMTM-2 SingleQuotsTM supplement (CC-4176), which was required for the growth of endothelial cells. For miRNA transfection, 4 × 10⁵ cells were plated in a 60 mm² culture dish and transfected with 60 nM miRNA mimics for 6 hours. Control miRNA and miRNA mimics were purchased from Bioneer (Bioneer, Daejeon, South Korea) and transfected using Lipofectamine^{™} RNAiMAX transfection reagent (Thermofisher, Cat# 13778150) according to the manufacturer's protocol. After 24 hours, the cells were harvested and transfection was assessed, and the results were illustrated in FIG. 7C.

As illustrated in FIG. 7C, it was identified that the VEGFA mRNA level was significantly decreased in HUVECs transfected with pre-miR-645 (p < 0.01), and overexpression of miR-205-5p and miR-646 decreased the expression level of VEGFA mRNA.

### 2.6 Effect of overexpression of miRNA on VEGFA protein expression

HUVECs were lysed using an RIPA buffer containing a protease inhibitor. Total 10 µg of a cell lysate was separated by SDS-polyacrylamide gel electrophoresis (Invitrogen, Cat# XP08162BOX) and transferred to a 0.45 µm nitrocellulose membrane (BIO-RAD, Cat# 1620115). After blocking, the membrane was incubated with each primary antibody overnight at 4°C, washed in TBS-t three times for 10 minutes, and then incubated with an appropriate secondary antibody for 1 hour at room temperature. The data were visualized using a chemiluminescence imaging system (VILBER, Fusion SOLO S), and the results were illustrated in FIG. 7D.

As illustrated in FIG. 7D, it was confirmed that overexpression of miR-645 in HUVECs decreased VEGFA protein levels.

In summary, it was confirmed that the levels of miRNAs (miR-205-5p, miR-645, and miR-646) in microvesicles were significantly increased in a patient group with cerebral infarction due to cancer-related coagulation disorders. Increased miRNAs in blood microvesicles may diagnose the occurrence of cerebral infarction due to coagulation disorders in cancer patients, and may be used as guideline markers in the application of anticoagulants in cancer patients. In addition, it was confirmed that miRNAs (miR-205-5p, miR-645, and miR-646) in microvesicles may promote angiogenesis and thus can be used as therapeutic agents for ischemic diseases.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A biomarker composition for use in diagnosing coagulation disorders comprising at least one miRNA selected from the group consisting of miR-205-5p, miR-645, and miR-646.

2. The biomarker composition for use in diagnosing coagulation disorders of claim 1, wherein the coagulation disorders are cancer-related coagulation disorders.

3. The biomarker composition for use in diagnosing coagulation disorders of claim 2, wherein the cancer-related coagulation disorder is cancer-related stoke.

4. The biomarker composition for use in diagnosing coagulation disorders of claim 1, wherein the miR-205-5p, miR-645, and miR-646 are miRNAs in extracellular vesicles.

5. The biomarker composition for use in diagnosing coagulation disorders of claim 4, wherein the extracellular vesicles are extracellular vesicles in blood.

6. The biomarker composition for use in diagnosing coagulation disorders of claim 4, wherein the extracellular vesicles are microvesicles.

7. The biomarker composition for use in diagnosing coagulation disorders of claim 1, wherein the miR-205-5p, miR-645, and miR-646 are highly expressed when coagulation disorders occur.

8. A composition for use in diagnosing coagulation disorders, the composition comprising an agent for measuring the expression level of at least one miRNA selected from the group consisting of miR-205-5p, miR-645, and miR-646.

9. The composition for use in diagnosing coagulation disorders of claim 8, wherein the agent is a primer or probe that specifically binds to at least one miRNA selected from the group consisting of miR-205-5p, miR-645, and miR-646.

10. A kit for use in diagnosing coagulation disorders, the kit comprising the composition for diagnosing coagulation disorders of claim 8.

11. A method for providing information for diagnosing coagulation disorders, the method comprising:
(a) isolating extracellular vesicles from a biological sample of a target; and
(b) comparing the expression level of at least one miRNA selected from the group consisting of miR-205-5p, miR-645, and miR-646 in the extracellular vesicles with the expression level of the corresponding miRNA in a comparative control sample.

12. The method for providing information for diagnosing coagulation disorders of claim 11, wherein in step (b) is determining that the target is coagulation disorders when the expression level of at least one miRNA selected from the group consisting of miR-205-5p, miR-645, and miR-646 is highly expressed compared to a comparative control group.

13. The method for providing information for diagnosing coagulation disorders of claim 11, wherein the biological sample is blood or serum.

14. A method for diagnosing and treating coagulation disorders, the method comprising:
(a) obtaining an analysis sample from a subject;
(b) diagnosing the subject with coagulation disorders when the expression level of at least one miRNA selected from the group consisting of miR-205-5p, miR-645, and miR-646 in the analysis sample of step (a) is highly expressed compared to a comparative control group; and
(d) administering a coagulation disorder therapeutic agent in an effective amount to the subject diagnosed with coagulation disorders of step (c).

15. The method for diagnosing and treating coagulation disorders of claim 14, wherein the coagulation disorder therapeutic agent is at least one selected from the group consisting of heparin, dalteparin, enoxaparin, tanzapirin, warfarin, xarelto, eliquis, pradaxa, and Lixiana.
